# EUROPÄISCHE PATENTSCHRIFT

(11) **EP 0 708 652 B1**
(45) Veröffentlichungstag und Bekanntmachung des Hinweises auf die Patenterteilung: **25.08.1999**
(21) Anmeldenummer: 95915783.5
(22) Anmeldetag: 11.04.1995
(51) Int. Cl.: A61K 35/78

(54) **PHYTOTHERAPEUTIKUM MIT VIRUSTATISCHER UND ANTIVIRALER WIRKUNG**
PHYTOTHERAPEUTIC AGENT HAVING A VIRUSTATIC AND ANTI-VIRAL EFFECT
AGENT PHYTOTHERAPEUTIQUE A ACTION VIROSTATIQUE ET ANTIVIRALE

(30) Priorität: 03.05.1994 DE 4415539
(43) Veröffentlichungstag der Anmeldung: 01.05.1996
(73) Patentinhaber: OMER, Osama L. M., Dr.Dr., 79618 Rheinfelden (DE)
(72) Erfinder: OMER, Osama L. M., Dr.Dr., 79618 Rheinfelden (DE)
(86) Internationale Anmeldenummer: DE9500513
(87) Internationale Veröffentlichungsnummer: WO9529688

(56) Entgegenhaltungen:
- WO-A-89/12454

## Beschreibung

Herpes Simplex Viren (HSV) gehören zur Gruppe der DNS-Viren. Von über 70 verschiedenen serologischen Subtypen sind sechs für die Erkrankungen an Menschen von Bedeutung, und zwar:
(1) Herpes Simplex Virus Typ 1 (HSV-1);
(2) Herpes Simplex Virus Typ 2 (HSV-2);
(3) Varicella-Zoster Virus (VZV);
(4) Cytomegalovirus (CMV);
(5) Epstein-Barr Virus (EBV);
(6) Herpes Virus Typ 6 (HHV-6).

Ein besonderes Merkmal dieser Virengruppen sind die häufigen Rezidive der Erkrankung, die durch Reaktivierung einer latent gebliebenen Infektion zustande kommen. Die Reaktivierung von HSV-1 Viren ist z.B. verantwortlich für den immer wiederkehrenden Herpes labialis und für Stomatitis aphtosa herpetica. Der HSV-2 Virus verursacht den rezividierenden lästigen Herpes genitalis und steht auch in Zusammenhang mit dem Cervix-Karzinom und der HIV-Erkrankung. Die infektiöse Mononukleose ist nur eine der durch Ebstein-Barr-Virus (EBV) verursachten Erkrankungen. Das Spektrum des EBV reicht von subklinischen Verlauf über monosymptomatische Formen bis zum Vollbild der Mononukleose. Dieser Virus siedelt sich manchmal in den Leberzellen an and äußert sich durch die Erhöhung von Transaminasen und Hepatitiden. Episodisch verlaufende depressive- und Angst-Erkrankungen werden in Zusammenhang mit dem EBV-Rezidiv gebracht. Der Cytomegalie-Virus (CMV) weist einen hohen Durchseuchungsgrad auf (30-40% der Bevölkerung). Bei Trägerinen des CMV kann es durch diaplazentäre Übertragung zu einer Mißbildung kommen. Außerdem führt dieser Virus bei den Patienten, die eine zytostatische Therapie z.B. nach einem Organ-Transplantat, oder bei Patienten mit einem geschwächten Immunsystem z.B. bei HIV Infekt, häufig zu tödlich verlaufenden Pneumonien, Myokarditis oder zu Hepatitis. Infektionen mit Hepatitis B und C Viren, die ebenfalls zu den DNS-Viren gehören, führen sehr häufig zu einer chronischen aktiven Hepatitis, die in Leberzirrhose und in Leberinsuffizienz endet. Allergische Rhino-Sino-Bronchitiden sind Krankheiten, die durch die Hyperreagibilität der Schleimhäute der Atmungsorgane und dadurch verursachten Schleimhautödemen gekennzeichnet sind. Man weiß, daß auch hier in vielen Fällen virale Infekte die allergische bzw. *"*asthmatische*"* Reaktionschwelle der Atemwege dauernd senken können.

Eine kausale Dauertherapie für diese Erkrankungen gibt es bisher nicht. Man behandelt sie entweder prophylaktisch (z.B. Immunglobuline) oder, wenn überhaupt noch möglich, rein symptomatisch. Eine Chemotherapie gegen HSV-Viren und Hepatitis B und Hepatitis C hat bis jetzt wenig Erfolge gezeigt; die Gründe dafür liegen unter anderem an der Cytotoxizität der neuentdeckten antiviralen Substanzen. Die bisher bekannten antiviralen Substanzen (z.B. Acyclovir) zeigen nur in der aktiven Vermehrungsphase eine virostatische Wirkung, jedoch nicht in der latenten Phase und sind somit nicht in der Lage das Virus auszurotten. Außerdem sind diese Behandlungen sehr Kostenaufwendig. So z.B. wird eine CMV-Erkrankung bei einem organtransplantierten und immungeschwächten Patient in einer Klinik in die USA ca. $10,000-$50,000 kosten. Für allergische Rhino-Sino-Bronchitiden bieten Kortikoide und Antihistaminika eine vorübergehende symptomatische Linderung. Auch diese Medikamente sind mit ernsthaften, manchmal lebensbedrohlichen Nebenwirkungen behaftet. Eine Arznei-Erfindung mit antiviraler und virustatischer Wirkung, die gleichzeitig risikofrei und preiswert ist, wäre sehr wünschenswert.

Die Pflanzen Wermutkraut (Artemesia absinthium), Mastixresin (Resin pistacia lenticus) und Frucht von Delphenium denudatum sind bekannte Drogen. Nicht bekannt sind ihre antivirale bzw. antiallergische Wirkung. Cardamonfrucht (Fructus elettaria cardamomum), Rosenblüten (Flores rosae) und Borretschblumen (Flores onosma brateatum boraginaceae) sind als Gewürzdrogen bzw. Schmuckdrogen bekannt. Die Erfindung beschreibt die antivirale bzw. virostatische und anti-allergische Wirkung von Wermutkraut, Mastixresin und Frucht von Delphinium denudatum einzeln und in Kombination untereinander sowie in Kombination mit Rosenblüten, Cardamom und Borretschblumen.

Um die gewünschte therapeutische antivirale bzw. virostatische und antiallergische Wirkung zu demonstrieren wurden nachfolgende Pflanzen bzw. deren Mischungen getestet:
Gruppe 1. Wermutkraut
Gruppe 2. Mastixresin
Gruppe 3. Delphinium denudatum Frucht
Gruppe 4. Wermutkraut und Mastixresin
Gruppe 5. Wermutkraut und Delphenium denudatum Frucht
Gruppe 6. Delphinium denudatum Frucht und Mastixresin
Gruppe 7. Wermutkraut, Mastixresin und Delphenium denudatum Frucht
Gruppe 8. Gruppe 7 + Rosenblüten
Gruppe 9. Gruppe 7 + Rosenblüten und Cardamomfrucht
Gruppe 10.Gruppe 7 + Rosenblüten, Cardamomfrucht und Borretschblumen.

Diese Pflanzen bzw. deren Mischungen werden getrocknet, mit UV-Licht desinfiziert, feingemahlen, versiebt (>1mm Größe) und in Verbindung mit üblichen Applikationsträgern als Salbe vorbereitet. Für die orale Verabreichung wurden sie in Gelatinkapseln abgefüllt, um ein Gesamtgewicht der Kapseln von 500 mg zu erhalten. Bei Mischungen werden die einzelnen Pflanzen bzw. Pflanzenteilen zu gleichen Teilen kombiniert, in Kapseln abgefüllt und oral verabreicht. Um einen Anhaltspunkt für die antivirale Aktivität zu bekommen, wurden Patienten die an einem rezidiven Herpes labialis erkrankten, mit den Pflanzen bzw. Pflanzengemisch entweder äußerlich oder innerlich behandelt. Herpes labialis ist die weitverbreitetste Krankheit die durch den Herpes Simplex Virus hervorgerufen wird. Sie ist eine typische rekurrierende Herpeserkrankung, die durch die verschiedensten Umstände provoziert wird (z.B. durch Grippalinfekte, Pneumonien, intensive Besonnung, Stressituationen oder gastrointestinale Störungen). Die einzelnen Effloreszenzen gleichen denen des Herpes Zoster. Ohne Behandlung trocknen die Bläschen an der Lippe nach zehn bis 14 Tagen ab. Die Diagnose wurde anhand des klinischen Erscheinungsbildes und der typischen Lokalisation der Bläschen und der Ulzeration gestellt. Vor der Aufnahme in die Studie wurde das Einverständnis des Patienten geholt. Eine virustatische Aktivität wurde angenommen, wenn bei einer frischen, nicht länger als 2-3 Tage alten Infektion die typischen Herpes-Bläschen innerhalb von 3-4 Tagen austrockneten oder eine deutliche Rückbildungstendenz zeigten. Der Nachweis antiviraler Wirkung war erbracht, wenn von einem Abstrich aus den Virusbläschen oder aus der Mundschleimhaut in Zellkulturen kein Viruswachstum stattfand.

Aus WO-A-8912454 geht hervor, daß eine Mischung aus Wermutkraut, Rosenblüten, Cardamomfrucht, Agarholt, Bambus-Manna, Mastixresin und Onosma-Blüten für die Behandlung von Depressionen geeignet ist.

Nun wurde herausgefunden, daß Wermutkraut, Mastixresin und Delphinium denudatum Frucht eine deutliche antivirale Aktivität besitzen. Weder die Rosenblüten noch die Cardamom Frucht noch die Borretschblumen besitzen alleine irgendwelche antivirale Aktivität. Sie entfalten aber in Kombination mit Wermutkraut, oder/und Mastixresin oder/und Delphinium donadatum Frucht eine synergistische Wirkung. Als besonders ausgewogen und optimiert zur Behandlung von Herpesinfektionen zeigen sich die Mischungen der Gruppen 7, 8, 9 und 10.

Die Erfindung wird anhand der folgenden Beispiele erläutert: Die einzelnen, unabhängig voneinander getrockneten, zu feinem Pulver gemahlenen und gesiebten Ausgangsmaterialien wurden miteinander gemischt, um das u.g. Erfindungsgemisch A bzw. Erfindungsgemisch B zu ergeben. Das Feinpulver wurde in Kapseln der Größe 00 abgefüllt, in denen das Gewicht der Mischung von 370 mg pro Kapsel erhalten wurde.

### Erfindungsgemisch A

| | |
|---|---|
| Wermutkraut | 180 mgm |
| Mastixresin | 70 mgm |
| Rosenblüten | 70 mgm |
| Cardamomfrucht | 50 mgm |
| Gesamtgewicht: | 370 mgm |

### Erfindungsgemisch B

| | |
|---|---|
| Wermutkraut | 180 mgm |
| Mastixresin | 70 mgm |
| Delphenium denudatum Frucht | 120 mgm |
| Gesamtgewicht | 370 mgm |

Folgende Versuche wurden mit dem Erfindungsgemisch A durchgeführt.

### Versuch 1:

Therapeutischer Einsatz des Erfindungsgemischs A bei der Behandlung von Stomatitis aphtosa herpetica.

Die häufigste Primärmanifestation der HSV-1 Infektion ist die Stomatitis aphtosa herpetica. Diese Erkrankung, die vorwiegend Kinder befällt, zeigt eine Tendenz zur spontanen Rückbildung der schmerzhaften Ulzerationen im Mund- und Schleimhautbereich zwischen dem achten und zehnten Tag. Die Diagnose wurde anhand des klinischen Erscheinungsbildes, der typischen Lokalisation der Ulzeration, HSV-1 Antigentiter (Latex Agglutinationtest) und der Nachweis von HSV-1 aus dem Abstrich von Mundschleimhaut gestellt. Vor der Aufnahme in der Studie wurde das Einverständnis des Patienten eingeholt. Eine antivirale Aktivität wurde angenommen, wenn bei einer frischen, nicht länger als 2 -3 Tage alten Infektion die Bläschen innerhalb von 3-4 Tagen austrockneten oder eine deutliche Rückbildungstendenz zeigten und nach 3-wöchiger Behandlung kein Viruswachstum aus dem Abstrich der Mundschleimhaut möglich war. Zehn Patienten mit dieser Erkrankung wurden erfolgreich behandelt. Keinerlei Nebenwirkungen wurden beobachtet.

### Versuch 2:

Therapeutischer Einsatz des Erfindungsgemischs A bei der Behandlung von Herpes genitalis.

Wie der Herpes labialis ist der Herpes genitalis eine typische rekurrierende Krankheit, die ebenfalls sehr weit verbreitet ist. Nach einer Schätzung leiden etwa 20 Millionen Amerikaner darunter. Ein akuter Schub dieser Erkrankung heilt ohne spezifische Therapie in zehn bis 14 Tagen ab. Die Diagnose wurde anhand des klinischen Erscheinungsbildes, der typischen Lokalisation der Ulzeration, HSV-1 Antigentiter (Latex Agglutinationtest, Werte höher als 1:4) gestellt. Vor der Aufnahme in der Studie wurde das Einverständnis des Patienten eingeholt. Eine antivirale Aktivität wurde angenommen, wenn bei einer frischen, nicht länger als 2-3 Tage alten Infektion die Bläschen innerhalb von 3-4 Tagen austrockneten oder eine deutliche Rückbildungstendenz zeigten und in den nachfolgenden 6-8 Monaten kein Rezidiv beobachtet wurde. Acht Patienten konnten erfolgreich behandelt werden. Keinerlei Nebenwirkungen wurden dabei beobachtet.

### Versuch 3:

Therapeutischer Einsatz des Erfindungsgemisches A bei der Behandlung von Herpes Zoster.

Herpes Zoster entsteht durch Reaktivierung einer latenten Varicella-Zoster Virusinfektion meist Jahre nach früher durchgemachten Windpocken. Das typische Erscheinungsbild von Zoster bereitet keine diagnostischen Schwierigkeiten; der Ausschlag bleibt typischerweise auf ein Dermatom beschränkt. Die schmerzhaften Bläschen heilen in zehn bis 14 Tagen - manchmal später - ab. Vor der Aufnahme der Studie wurde das Einverständnis des Patienten eingeholt. Eine antivirale Aktivität wurde angenommen, wenn bei einer frischen, nicht länger als 2 -3 Tage alten Infektion die Bläschen innerhalb von 3-4 Tagen austrockneten oder eine deutliche Rückbildungstendenz zeigten und die Patienten nach 21-tägiger Behandlung Schmerzfrei waren. Zehn Patienten wurden mit der Erfindung erfolgreich behandelt. Es wurden keine Nebenwirkungen beobachtet.

Folgende Versuche wurden mit dem Erfindungsgemisch B durchgeführt:

### Versuch 1

Therapeutischer Einsatz des Erfindungsgemisches B bei der Behandlung von infektiöser Mononukleose (EBV-Infekt).

Die infektiöse Mononukleose wird durch den Epstein-Barr Virus verursacht, der ebenfalls der Gruppe der Herpesviren angehört. Er ist mit größter Wahrscheinlichkeit als Ursache beteiligt bei der Entstehung des Burkitt-Lymphoms, des nasopharyngealen Karzinoms und der immer wiederkehrenden ängstlichen, depressiven Krisen mit autonomen Störungen. Fieber, Halsschmerzen und starke Schwellungen des zervikalen Lymphknotens sind bei jeder akuten Mononukleose vorhanden. In diagnostischer Hinsicht ist bei der akuten Mononukleose der Blutausstrich charakteristisch. Es besteht eine Lymphomonozytose mit zehn Prozent atypischen T-Lymphozyten. Der Anteil dieser T-Lymphozyten kann bis zu 40 Prozent steigen. T-Lymphozyten sind große Zellen mit unterschiedlich intensiv gefärbtem Plasma und verschieden geformten Kernen. Von diagnostischer Bedeutung ist außerdem der Nachweis von Antikörpern gegen das Kapsid des Epstein-Barr Virus und der Paul-Bunnell-Test. Als diagnostische Auswahlkriterien wurden das typische klinische Bild, das Bild des typischen Blutausstrich bei Mononukleose, Paul-Bunneltest und der Nachweis von Antikörpern gegen das Kapsid des EBV herangezogen. Vor der Aufnahme in der Studie wurde das Einverständnis des Patienten eingeholt. Eine antivirale Aktivität wurde angenommen, wenn bei einer frischen, nicht länger als 2 -3 Tage alten Infektion die klinischen Symptome und Blutchemischen Werte eine deutliche Rückbildungstendenz zeigten und wenn kein CMV in Blut, Urin oder Rachenabstrich nachgewiesen werden konnte. Fünf Patienten wurden erfolgreich behandelt.

### Versuch 2

Therapeutischer Einsatz des Erfindungsgemisches B bei de Behandlung von CMV Infizierten Patienten.

Die Erkrankung durch CMV verursacht Schädigung von Lunge, Leber und Urogenitaltrakt. Die Übertragung erfolgt vorwiegend über den Urogenitaltrakt. Infolge des hohen Durchseuchungsgrades der Zytomegalie ist die Komplimentbindungsreaktion, die lebenslänglich positiv bleiben kann, alleine von geringer diagnostischer Bedeutung. Als diagnostische Auswahlkriterien dienten: Positive Komplimentbindungsreaktion, erhöhte Gamma-GT Werte, erhöhte Gamma-Globuline und der spezifische Nachweis von IgG-CMV. Akut CMV erkrankte Patienten sowie die Kinder wurden nicht in die Studie aufgenommen. Vor der Aufnahme der Studie wurde das Einverständnis des Patienten eingeholt. Eine antivirale Aktivität wurde angenommen, wenn die pathologischen Blut- und Leberwerte eine deutliche Rückbildungstendenz zeigten und wenn kein CMV in Blut, Urin oder Rachenabstrich nachgewiesen werden konnte. Acht Patienten wurden in dieser Gruppe erfolgreich behandelt. Die Behandlungdauer Betrug im Durchschnitt 3 Monate. Es wurden keine Nebenwirkungen beobachtet.

### Versuch 3

Therapeutischer Einsatz des Erfindungsgemisches B bei der Behandlung von chronisch aktiver Hepatitis.

Verdacht auf chronisch aktive Hepatitis besteht bei aktiver Hepatopathie: erhöhte Transaminase von über 6 Monaten bei Fehlen exogener Noxen (z.B. Alkohol). Für die Aufnahme in der Studie wurden folgende Auswahlkriterien erfüllt: Chronisch erhöhte Transaminase, Nachweis von HB- und Hc-Antigenen und eine pathologische Elektrophorese mit Erhöhung der Gamma-Globuline und der Gesamteiweisswerte sowie durch eine Leberbiopsie histologisch gesicherte Diagnose einer chronisch aktiven Hepatitis. Als Wirkungskriterien der Erfindung dienten die Normalisierung oder zumindest der Trend zur Normalisierung der Gamma-Globuline und Leber-Transaminasen. Fünf Patienten mit Hepatitis B und 9 mit Hepatitis C wurden erfolgreich behandelt.

### Versuch 4

Therapeutischer Einsatz des Erfindungsgemisches B bei der Behandlung von chronischen allergischen Rhino-Sino-Bronchitiden.

Da die chronischen allergischen Rhino-Sinu-Bronchitiden keine einheitliche Pathogenese haben, wurde die Diagnose anhand des klinischen Bilds und der Anamnese gestellt. Vor der Aufnahme in der Studie wurden folgende diagnostische Befunde erhoben:
1. Gezielte Allergie-, Medikamenten- und Atopie-(Familien)Anamnese.
2. Lungenfunktionsprüfung (VK und FEV1)
3. Blutbild und BSG, Elektrophorese
4. Menge und Dauer der Einnahme von Kortison-Präparaten.

Die Wirksamkeit der Erfindung wurde von der Normalisierung der am Baseline erhobenen pathologischen Befunde, der Reduzierung des Kortison-Bedarfs und der Symptomfreiheit in der Follow-up Periode von 1 bis 2 Jahren abhängig gemacht. Fünfzehn Patienten wurden erfolgreich behandelt. Der Wirkungseintritt war erst nach 2 Wochen zu beobachten, dafür war sie langanhaltend.

Anhand der oben aufgeführten Versuche ergeben sich folgende Zielgruppen für die Verwendung nach der Erfindung:
1. Patienten mit einer Infektion durch DNS-Viren der Herpes Familien, bei denen sich die Herpeskrankheit von Zeit zu Zeit in einer bunten Vielfalt von klinischen Erscheinungen äußert, die von Haut- und Schleimhautbefall bis zur generalisierten Allgemeininfektion reicht.
2. Patienten mit chronischer aktiver Hepatitis deren Ursache DNS-Hepatitis B oder Hepatitis C Viren sind.
3. Patienten mit chronischen rezidivierenden allergischen Rhino-Sino-Bronchitiden.

## Patentansprüche

1. Die Verwendung von einer oder mehreren der folgenden Pflanzen bzw. Pflanzenteile in Trockenpulver- oder Extraktform und in Verbindung mit den üblichen Applikationsträgern zur Herstellung eines Arzneimittels für die Behandlung von Infektionen, die durch DNS-Viren der Herpes Familie oder durch DNS-Hepatitis B oder durch Hepatitis C Viren verursacht werden, sowie zur Behandlung von chronischen allergischen Rhino-Sinu-Bronchitiden:
Wermutkraut (Artemisia absinthium)
Mastixharz (Resin pistacia lenticus)
Delphinium denudatum Frucht

2. Die Verwendung nach Patentanspruch 1, wenn dazu Rosenblüten, und/oder Cardamomfrucht und/oder Borretschblumen (flores onosma brateatum boraginaceae) zugegeben wird.

## Claims

1. An application of one or more of the following herbs or part of the herbs, in the form of dried powder or extract and in association with the usual carrier substances for the preparation of a medicinal product, for treating infections caused by DNA virus of the herpes family or DNA hepatitis B or hepatitis C viruses, or suffering from chronic allergic rhino-sino-bronchitis:
wormwood (artemisia absinthium)
resin of mastic (resin pistacia lenticus)
fruit of delphinium denudatum

2. An application according to claim 1, wherein buds of roses and/or cardamom fruit and/or borage flowers (flores onosma brateatum boraginaceae) are added.

## Revendications

1. L'utilisation d'une des plantes suivantes ou de plusieurs des plantes suivantes respectivement des parties des plantes suivantes en forme d'extrait ou en poudre sèche combinées avec des supports classiques appropriés pour la fabrication des médicaments, pour le traitement des infections, causées par des virus de DNS de la famille d'herpès ou par des virus de DNS de l'hépatite B ou C et aussi pour le traitement des chroniques rhino-sino-bronchites allergiques:
herbes d'absinthe (artemesia absinthium)
résine de mastic (résine pistacia lenticus)
fruit de delphinium denudatum

2. L'utilisation en conformité avec les revendications sous no. 1, quand on ajoute des pétales de rose ou/et fruit de cardamom ou/et fleurs de borage (flores onosma brateatum boraginaceae).
